(19) Europäisches Patentamt
European Patent Office
Office européen des brevets

(11) **EP 1 886 629 A1**

(12) **EUROPEAN PATENT APPLICATION**
published in accordance with Art. 153(4) EPC

(43) Date of publication:
**13.02.2008 Bulletin 2008/07**

(51) Int Cl.:
***A61B 6/00*** (2006.01)

(21) Application number: **06746341.4**

(22) Date of filing: **12.05.2006**

(86) International application number:
**PCT/JP2006/309544**

(87) International publication number:
**WO 2006/129462 (07.12.2006 Gazette 2006/49)**

(84) Designated Contracting States:
**DE FR GB**

(30) Priority: **01.06.2005 JP 2005161674**
**20.09.2005 JP 2005272128**

(71) Applicant: **Konica Minolta Medical & Graphic, Inc.**
**Tokyo 163-0512 (JP)**

(72) Inventors:
• **SHINDEN, Yuko**
**Konica Minolta Medical & GraphicInc**
**Tokyo 1630512 (JP)**

• **OHARA, Hiromu**
**Konica Minolta Medical & GraphicInc**
**Tokyo 1630512 (JP)**
• **UMEKI, Mamoru**
**Konica Minolta Medical & GraphicInc**
**Tokyo 1630512 (JP)**

(74) Representative: **Henkel, Feiler & Hänzel**
**Patentanwälte**
**Maximiliansplatz 21**
**80333 München (DE)**

(54) **DIGITAL RADIATION IMAGE CAPTURING SYSTEM**

(57) A digital radiation image imaging system to detect an edge-emphasized image highly accurately, and to output the detected edge-emphasized image without deteriorating the image is provided.

Under the assumption that D ($\mu$m) represents a focal point diameter of X-ray tube, S ($\mu$m) represents a minimum control unit of a digital detector, A ($\mu$m) represents a minimum control unit of an image output apparatus, R1 (m) represents a distance from a focal point of X-ray tube to a subject, R2 (m) represents a distance from a subject to the digital detector, magnifying power M equals (R1 + R2) / R1, and E represents an edge emphasis width caused by X-ray refraction, S > A and D $\geq$ (S - E) / (M - 1) are caused to hold, and the minimum control unit of the digital detector S and an aggregate of n pieces of minimum control units of the image output apparatus are caused to correspond as data to be reproduced and outputted.

FIG. 2 (a)

X-RAY TUBE 13
SUBJECT 11
DIGITAL DETECTOR 10
DIGITAL DETECTOR 10
R1
R2
FOCAL POINT a
CONTACT IMAGE
CONTACT IMAGING POSITION
PHASE CONTRAST (ENLARGEMENT) ENLARGEMENT IMAGING POSITION
PHASE CONTRAST (ENLARGEMENT) ENLARGEMENT IMAGING 12

# FIG. 2 ( b )

DIGITAL DETECTOR 10

PIXEL

PIXEL

S

S

**Description**

**FIELD OF THE INVENTION**

The present invention relates to a digital radiation image generating system that images a subject on a digital basis through a phase contrast imaging method representing one of enlargement radiography, then, minifies digital image data (enlarged image of the subject) thus obtained to output them so that the image data may agree with a style of diagnosis (diagnosis based on an image whose size is substantially the same as that of the subject (which is called a life-size)) of a doctor for X-ray interpretation.

An image taken through a phase contrast imaging method is an image with high visibility wherein a focus marginal section (contours) are subjected to edge-emphasis, and it is highly expected in a medical field, and an improvement in diagnosis accuracy in a mammography (breast images) field is expected.

**TECHNICAL BACKGROUND**

Even in the field of medical images, the digitalization is advancing, and in the field of digital image radiographing, spatial resolution for reading images is restricted, depending on a reading pixel size of an X-ray detector or on a size of a reading sampling pitch. In this case, there are problems that a subject smaller than the reading pixel size or than the reading sampling pitch cannot be described and that a contour of the subject described turns out to be blurred even in the case of a subject larger than the reading pixel size or than the reading sampling pitch.

In addition, an X-ray detector becomes minute and complicated in terms of a structure, and a volume of data to be handled is increased, resulting in troubles that costs of memory for an X-ray detector and for data processing are increased and time required for data processing is increased.

Although the reading pixel size or the reading sampling pitch is important, it is impossible to achieve an improvement of visibility of a marginal section (contour) subjected to edge-emphasis in an image (image used for diagnosis) to be presented finally to a doctor for X-ray interpretation, even if miniaturization alone for the reading pixel size or the reading sampling pitch is attempted.

Patent Document 1 discloses a structure wherein visibility of an edge-emphasized marginal section (contour) can be maintained by keeping an output pixel size and each of the reading pixel size and the reading sampling pitch to be in a prescribed relationship, in a phase contrast imaging method.

For example, when conducting phase contrast imaging by using CR (Computed Radiography) or FPD (Flat Panel Detector), and when outputting the obtained image data on a film or a viewer, a reading pixel and an output pixel can be made to be on a one-to-one correspondence, if $B = A/M$ holds under the assumption that M represents an imaging magnification (magnifying power) in phase contrast imaging, A represents a minimum control unit (pixel size) in the case of reading, and

B represents a minimum control unit (pixel size) in the case of outputting. In this case, a part of the edge-emphasized marginal image is not lost in the course of interpolation processing, because minifying interpolation processing is not needed, and image deterioration is not caused, which is preferable and is known.

[Patent Document 1] Japanese Patent Publication Open to Public Inspection No. 2001-311701]

**DISCLOSURE OF THE INVENTION**

**SUBJECT TO BE SOLVED BY THE INVENTION**

In order to output an image in which an edge portion of marginal section (contour) of a specific region, being different from peripheral areas, is emphasized, as one of features of the image captured by employing the phase contrast imaging method, onto a film or a viewer with good visibility, it is necessary to faithfully output the image without losing the details of the edge-emphasized marginal images.

The present invention is achieved on the basis of the abovementioned demands. An object of the present invention is to provide a digital radiation image imaging system, which makes it possible not only to detect an edge-emphasized image in a high accurate mode, but also to output the edge-emphasized image detected without deteriorating its image quality.

**MEANS FOR SOLVING THE SUBJECT**

A digital radiation image imaging system of the present invention is characterized in that, in the digital radiation image imaging system, which includes: an X-ray tube to irradiate ,X-rays onto a subject; a digital detector to detect X-rays penetrated through the subject; an image outputting apparatus to output an X-ray image detected by the digital detector, so as to conduct a phase contrast imaging operation, a minimum control unit of the digital detector, represented by "S" ($\mu$m), and a focal point diameter of the X-ray tube, represented by "D" ($\mu$m), fulfill Equations indicated as follows:

$$S > A,$$

and

$$D \geq (S - E) / (M - 1),$$

where A ($\mu$m): minimum control unit of the image outputting apparatus,
R1 (m): distance from a focal point of the X-ray tube to the subject,
R2 (m): distance from the subject to the digital detector,
M: magnifying power defined by an Equation of

$$M = (R1 + R2) / R1,$$

and
E: edge emphasis width formed by deflections of the X-rays, and
the minimum control unit "S" of the digital detector and an aggregate of "n" peaces of the minimum control unit "A" of the image outputting apparatus are reproduced and outputted while correlating them with each other as data.

Further, another digital radiation image imaging system of the present invention is characterized in that, in the digital radiation image imaging system, which includes: an X-ray tube to irradiate X-rays onto a subject; a digital detector to detect X-rays penetrated through the subject; an image outputting apparatus to display an X-ray image detected by the digital detector, so as to conduct a phase contrast imaging operation, a minimum control unit of the digital detector, represented by "S" ($\mu$m), and a focal point diameter of the X-ray tube, represented by "D" ($\mu$m), fulfill Equations indicated as follows:

$$S > A,$$

and

$$D \geq S / (M - 1),$$

where A ($\mu$m) : minimum control unit of the image outputting apparatus,
R1 (m): distance from a focal point of the X-ray tube to the subject,
R2 (m): distance from the subject to the digital detector,
M: magnifying power defined by an Equation of

$$M = (R1 + R2) / R1,$$

and
E: edge emphasis width formed by deflections of the X-rays, and
the minimum control unit "S" of the digital detector and an aggregate of "n" peaces of the minimum control unit "A" of the image outputting apparatus are reproduced and outputted while correlating them with each other as data.

**EFFECT OF THE INVENTION**

According to the present invention, since the radiographing conditions at the time of the phase contrast imaging operation are established so as to detect the edge-emphasized image highly accurately, and the reading control unit and the aggregate of the outputting control units are correlated with each other in an appropriate relationship, it becomes possible not only to suppress the image deterioration, but also to improve the visibility of the edge-emphasized image concerned.

**BRIEF DESCRIPTION OF THE DRAWINGS**

Fig. 1 shows a brief configuration of a digital radiation image imaging system embodied in the present invention.
Fig. 2(a) and Fig. 2(b) show brief configurations of X-ray imaging apparatus embodied in the present invention.
Fig. 3 shows an explanatory drawing for explaining a principle how to generate a phase contrast edge-emphasis of a subject image due to deflections of X rays.
Fig. 4 shows a graph indicating a half width value of the phase contrast edge-emphasis.
Fig. 5 shows a graph indicating a half width value of the phase contrast edge-emphasis when employing a Coolidge X-ray tube.
Fig. 6 shows an explanatory drawing for explaining a fact that a pixel size of a digital detector is detectable even if it has a predetermined dimension.
Fig. 7(a) and Fig. 7(b) show explanatory drawings for explaining a control unit.
Fig. 8 shows an explanatory drawing for explaining a fact that, due to geometric unsharpness, an edge-emphasis width is widened to such a width that is wider than that created by X rays emitted from an ideal point light source.
Fig. 9 shows a drawing indicating a relationship between a minimum control unit (reading pixel size, or reading sampling pitch) "S" at a digital detector and another minimum control unit (output pixel size, or output writing pitch) "A" of an image output apparatus.
Fig. 10 shows a brief configuration of a digital radiation image imaging system embodied in the present invention as the second embodiment.
Fig. 11 shows an internal configuration of a JOB manager shown in Fig. 10.
Fig. 12 shows an example of output setting table in which output setting information of each of output apparatuses shown in Fig. 10 are stored.
Fig. 13 shows a flowchart for explaining a flow of output controlling operations to be implemented by the JOB manager.
Fig. 14 shows a drawing indicating a relationship between an enlarged image and its output image, when the enlarged image and a minimum output unit are different from each other.

**EXPLANATION OF THE NOTATIONS**

1 an X-ray imaging apparatus

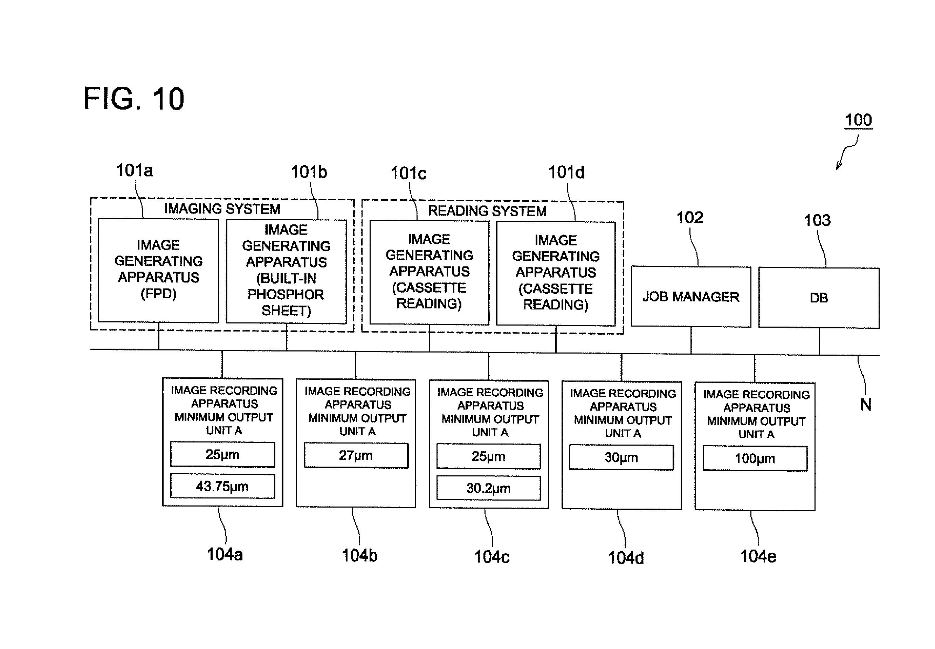

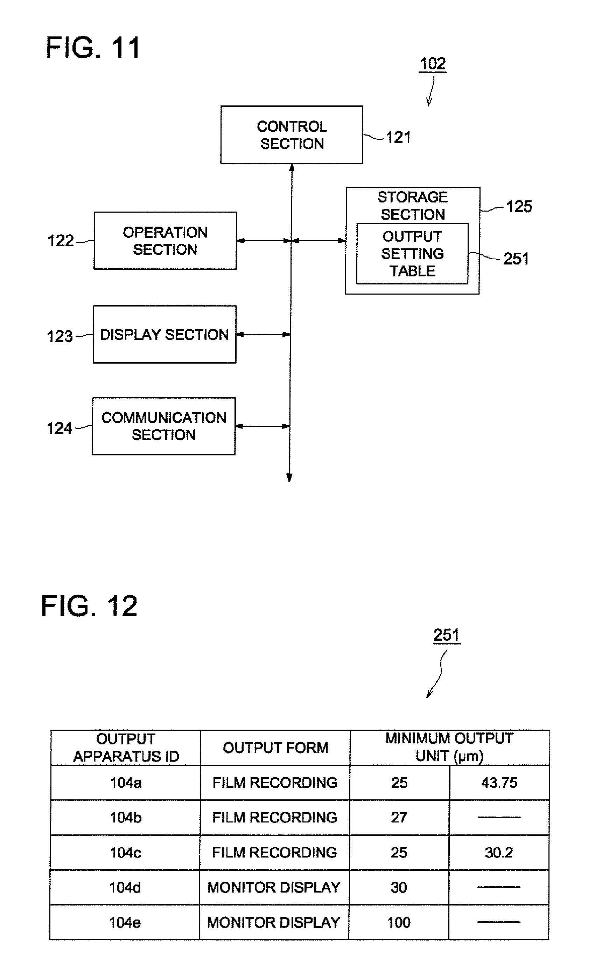

Fig. 12 shows an example of output setting table in which output setting information of each of output apparatuses shown in Fig. 10 are stored.

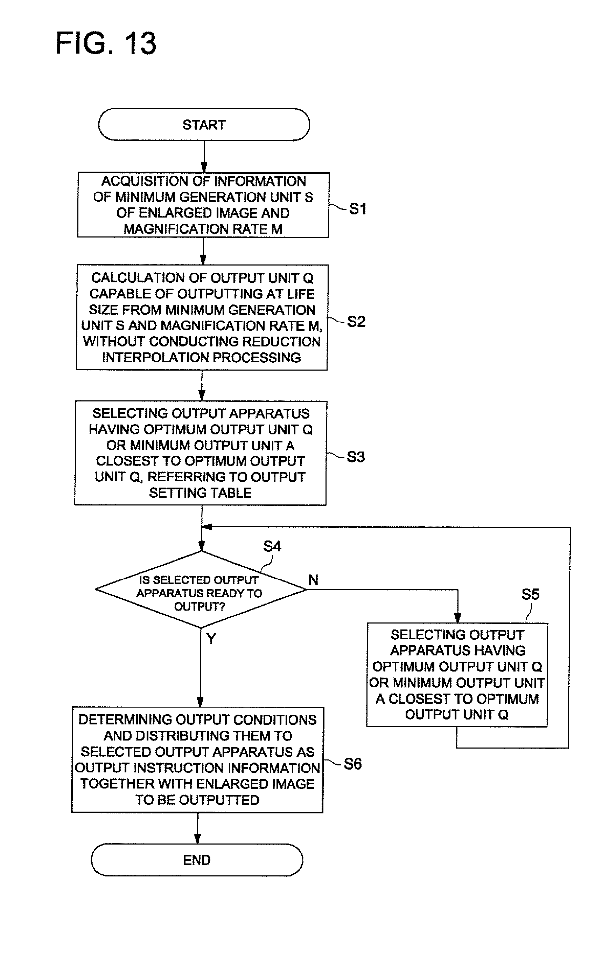

Fig. 13 shows a flowchart for explaining a flow of output controlling operations to be implemented by the JOB manager.

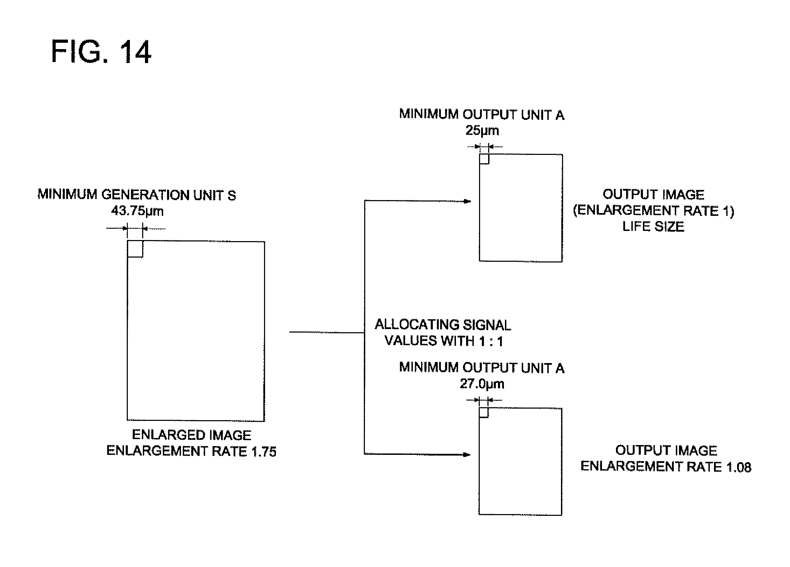

Fig. 14 shows a drawing indicating a relationship between an enlarged image and its output image, when the enlarged image and a minimum output unit are different from each other.

**EXPLANATION OF THE NOTATIONS**

| 1 | an X-ray imaging apparatus |
|---|---|
| 2 | an image processing apparatus |
| 3 | an image-print output apparatus |
| 4 | an image output apparatus |
| 4a | a viewer |
| 4b | a printer |
| 6 | a preservation apparatus |

| 100 | a digital radiation image imaging system |
| 101a - 101d | image generating apparatuses |
| 102 | a JOB manager |
| 103 | a DB |
| 104a - 104c | image recording apparatuses |
| 104d - 104e | image displaying apparatuses |

**BEST MODE FOR IMPLEMENTING THE INVENTION**

(First Example)

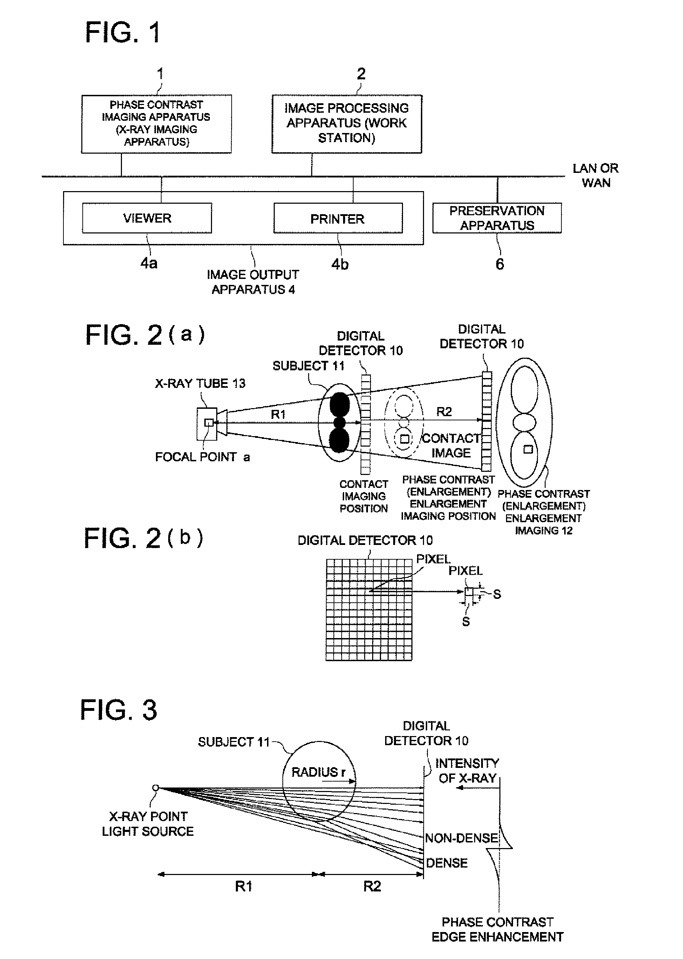

Fig. 1 shows a digital radiation image imaging system relating to the first embodiment. In the digital radiation image imaging system in the present embodiment, phase contrast imaging apparatus (X-ray imaging apparatus) 1, image processing apparatus (work station) 2, image output apparatus 4 (viewer 4a, printer 4b) and preservation apparatus 6 are connected each other through LAN or WAN. Each apparatus is made to be possible for communication corresponding to DICOM protocol, and it reproduces digital image data generated by a phase contrast imaging method.

First, in phase contrast imaging apparatus 1, imaging for two-dimensional flat digital image is conducted, and after an X-ray image is taken through the aforesaid imaging, image signals are taken out and image processing is conducted by image processing apparatus 2. Further, image signals are subjected to image-display on viewer 4 of image output apparatus 4, and are subjected to image-print output with printer 4b.

The phase contrast imaging apparatus 1 is preferably the so-called digital image imaging apparatus, and it is provided with digital detectors such as CR, FPD and a division type X-ray detector. It may further be one that is taken through imaging on a screen or a film, and a film after developing is digitized by a digitizer.

FPD includes two types including the so-called a direct type and an indirect type, and these types are not restricted in the present invention. In the direct type FPD, X-ray are irradiated on a-Se, and electric charges thus generated are collected to be accumulated temporarily in a capacitor. Then, the accumulated electric charges are taken out in order on a two-dimensional basis, to be made image signals finally.

The division type X-ray detector includes one wherein image signals are taken out by causing CCD to touch directly a scintillator with a plane that emits visible light when it is irradiated by X-ray, one wherein image signals are taken out by collecting emitted light with a glass fiber and by leading it to CCD or the one wherein image signals are taken out by introducing emitted light to CCD by the use of a lens.

A reading pixel size or a reading sampling pitch of the digital detector is defined to be minimum control unit S ($\mu$m) in the case of reading. For the minimum control unit S, $10 = S = 200 \mu$m is preferable. If S is greater than 200 $\mu$m, it is difficult to acquire precisely X-ray image transmitted through a subject, while, if it is smaller 10 $\mu$m, an yield rate is worsened and manufacturing cost is increased. More preferable is $30 = S = 100 \mu$m, and by conducting reading sampling in this area, it is possible to read without lacking an edge-emphasized boundary image obtained through phase contrast imaging, and sharpness is improved.

Viewer 4a is used to display imaged data and to check the quality of positioning of imaging region. When it is satisfactory, an engineer transmits the aforesaid data to preservation apparatus 6 such as a storage apparatus to preserve them. Further, simultaneously with this, the aforesaid image data may also be transmitted to an unillustrated work station for a doctor for X-ray interpretation.

A cathode ray tube (CRT), a liquid crystal, a plasma-display, a liquid crystal projector and organic EL can be used as the viewer 4a. In the viewer 4a, luminance: 400 - 1000 cd/m$^2$, contrast ratio: 200 - 10000, and depth of information: 8 or 16 bit are preferable. Though a size of an image plane is not restricted in particular, a size that can cover the whole of the region to be imaged is preferable. It is preferable that the name of a patient, a magnification rate for imaging and character information such as a date of imaging are displayed together with images. Further, the past image, other modality images such as X-ray CT and MRI, resection test body images and color images such as fundus images may also be displayed simultaneously or separately.

An output pixel size of the viewer 4a or an output writing pitch of printer 4b is defined to be minimum control unit A ($\mu$m) in the case of outputting.

Next, a phase contrast imaging method will be explained based on Fig. 2. Fig. 2 shows an outline of X-ray imaging apparatus 1 shown in Fig. 1. Contact imaging means imaging under the state where subject 11 is in contact with digital detector 10 or with a member including the digital detector 10. A distance from the position of subject 11 on the digital detector 10 side to the digital detector 10 or to the member including that is defined to be R2. The contact imaging means that R2 is 0 or it is substantially 0. A meaning of the expression that R2 is substantially 0 is that R2 is not more than 0.05 m, or magnifying power M is less than 1.1. The magnifying power M is defined to be a value obtained by dividing the maximum length of a projected image by a length of a corresponding portion of a main body of the subject.

A phase contrast image is obtained by selecting the magnifying power M of $1 < M = 10$. A range of $1.4 = M =$

3 is preferable, and by selecting the magnifying power M of this range, a phase contrast image having high image quality usable as an image for diagnoses can be obtained.

When digital detector 10 is installed to be away from subject 11 as shown in Fig. 2, it is possible to obtain phase contrast image 12 through imaging by X-rays emitted from X-ray tube 13. An occasion where R2 exceeds 0.05 m and an occasion where the magnifying power M is not less than 1.1 mean the phase contrast imaging. A range of distance R1 between focal point a of X-ray tube 13 of the present embodiment and subject 11 is 0.15 = R1 = 5 m when a form of an ordinary imaging room (especially, a distance between a floor and a ceiling) and a thickness of a subject are taken into consideration, and it is preferably 0.25 = R1 = 2 m when image quality and work efficiency are further considered. Further, a range of distance R2 between subject 11 and digital detector 10 subject 11 is 0.15 = R2 = 5 m when a form of an ordinary imaging room (especially, a distance between a floor and a ceiling) and image quality that makes a diagnosis possible are taken into consideration, and it is preferably 0.5 = R2 = 2 m.

A rotation anode hot-cathode tube is preferable as X-ray tube 13. Namely, in the rotation anode hot-cathode tube, an electron is emitted from a filament, then, the electron hits the anode on which optional voltage in a range from 10 kV to 500 kV is applied, and kinetic energy of the electron is converted into an electromagnetic wave to be emitted as X-ray. In this case, a carbon nanotube may be used as one that emits electrons, although a filament may also be used. It is preferable that the anode is made of a metal of molybdenum or tungsten, and it is rotated so that it may not be damaged by generation of heat caused by a collision of a thermoelectron. A form of the portion where the thermoelectron hits the anode is usually designed to be a perfect square when it is viewed in the direction of the emission, and it is called a focal point. A length of one side of this perfect square is called focal point diameter D which indicates a size of an X-ray source. The focal point diameter D is one to be shown generally by a manufacturer of X-ray tube as a specification, and it can be measured by using a pin-hole camera or a test chart as is established in JIS Z4702.

A range of the focal point diameter D is 1 = D = 300 μm and it is preferably 30 = D = 100 μm. By selecting the focal point diameter D of 1 μm or more, an output of X-ray just for being transmitted through subject 11 is obtained, and an image having high image quality suitable for a diagnosis can be obtained by selecting the focal point diameter D of 30 μm or more. If the focal point diameter is small, a period of time for imaging is long although image quality is improved. Since a form of a structure having a size of about 100 μm needs to be observed in the case of mammography, the focal point diameter is in a range from 30 μm to 100 μm because a smaller focal point is desired. By selecting the focal point diameter D in a range of 30 = D = 100 μm, an edge image which can be detected by digital detector 10 can be obtained, and an image with high sharpness can be obtained. A range of minimum control unit S (μm) representing a reading pixel size of digital detector 10 or a reading sampling pitch is 10 = S = 200 μm and it is preferably 30 = S = 100 μm. The smaller the minimum control unit S is, the more precise image is obtained and details of the structure can be observed. However, manufacturing of the detector becomes difficult, and an yield rate of products is lowered. It is preferable that an area of detection by digital detector 10 covers the total area where a subject region is enlarged.

A range of minimum control unit A representing an output pixel size of image outputting apparatus 4 or an output writing pitch is 25 = A = 300 μm, and when the minimum control unit A is too large, a contour of an image is blurred, while, when it is smaller, a precise image can be displayed, and it is possible to observe up to details of the structure. However, when the minimum control unit A is small, a manufacturing yield is worsened and manufacturing cost is increased. Further, an amount of image data grows greater, and it takes longer time for display and switching of images, resulting in a decline of work efficiency.

The preferable range is 50 = A = 200 μm, and when 200 μm is exceeded, it sometimes is difficult to diagnose in the case of precise diagnosis such as observing a minute structure.

Phase contrast imaging apparatus 1 is an apparatus that conducts imaging in the aforesaid method, and utilizes a phenomenon wherein an edge is generated on the circumference of an image of a subject by refraction of X-ray, to acquire a radiation image having higher sharpness.

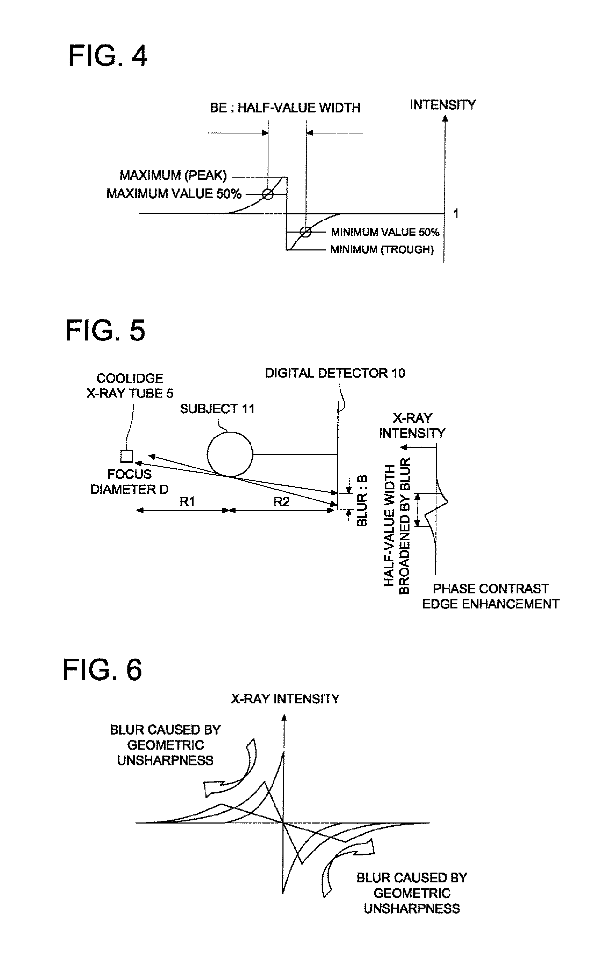

In the phase contrast imaging, an X-ray passing through the circumference of subject 11 is refracted and overlaps with X-ray having passed through a flank of subject 11 on digital detector 10, outside the circumstance of subject 11, as shown in Fig. 3, and intensity of X-ray is strengthened. On the contrary, in the vicinity of the inside of the circumstance of subject 11, X-ray intensity is weakened. As stated above, with respect to the X-ray intensity, a peak is caused on the outside and trough is caused in the inside, with the circumference of subject 11 serving as a boundary, thus, an edge is emphasized. This edge-emphasis function is also called an edge effect. Owing to this edge-emphasis function, an X-ray image having excellent sharpness whose circumstance is described clearly can be obtained. In this case, as shown in Fig. 4, if the X-ray source is regarded as a point source of light, half-width E of phase contrast edge emphasis can be expressed by the following expression (1);

$$E = 2.3 \ (1 + R2/R1)^{1/3} \ \{R2\delta \ (2r)^{1/2}\}^{2/3} \qquad (1)$$

wherein, δ represents a refractive index difference at the portion where X-ray is refracted and r represents a radius of an object (a subject).

On the other hand, in the medical job site and in the nondestructive inspection facility, Coolidge X-ray tube 5 (which is also called thermoelectron X-ray tube) is widely used. Fig. 5 shows an occasion where Coolidge X-ray tube 5 is used. In the Coolidge X-ray tube 5, a thermoelectron hits the anode made of metal such as tungsten to emit X-ray, and X-ray is emitted radially from a window in a form of a perfect square which is called a focal point. A length of one side of this window in a form of a perfect square is called focal point diameter. When using Coolidge X-ray tube 5, an X-ray source cannot be regarded as an ideal point light source. Namely, by a focal point serving as an X-ray source having a finite size, half-width E for phase contrast edge emphasis is broadened by the so-called geometric unsharpness as shown in Fig. 6, and intensity is decreased. In this case, half-width E for phase contrast edge emphasis can be expressed by the following expression (2);

$$EB = 2.3 \ (1 + R2/R1)^{1/3} \ \{R2\delta \ (2r)^{1/2}\}^{2/3} + D \ (R2/R1) \quad (2)$$

wherein D represents a focal point diameter of Coolidge X-ray tube 5 to be used.

When using Coolidge X-ray tube 5, half-width E for phase contrast edge emphasis is broadened by the so-called geometric unsharpness, and an edge emphasis image is blurred. However, on the contrary, detection of an edge emphasis image is possible even when a pixel size of digital detector 10 is relatively large, because half-width E is broadened.

For providing a highly sharp image obtained by phase contrast imaging as an image for diagnosis, an edge-emphasized image needs to be detected accurately firstly, and image information of the detected edge-emphasized image needs to be outputted under the diagnosable state without being lost secondly.

Under the assumption that "D" (μm) represents a focal point diameter of X-ray tube, "S" (μm) represents a minimum control unit (reading pixel size or reading sampling pitch) of a digital detector, "A" (μm) represents a minimum control unit (output pixed size or output writing pitch) of an image outputting apparatus, "R1" (m) represents a distance from a focal point of X-ray tube to a subject, "R2" (m) represents a distance from a subject to a digital detector, magnifying power "M" equals (R1 + R2) / R1 and "E" represents an edge emphasis width caused by X-ray refraction, S > A and D = (S - E) / (M - 1) are caused to hold, and the minimum control unit of the aforesaid digital detector and an aggregate of n pieces of minimum control units of the aforesaid image output apparatus are caused to correspond as data to be reproduced and outputted, in the present embodiment.

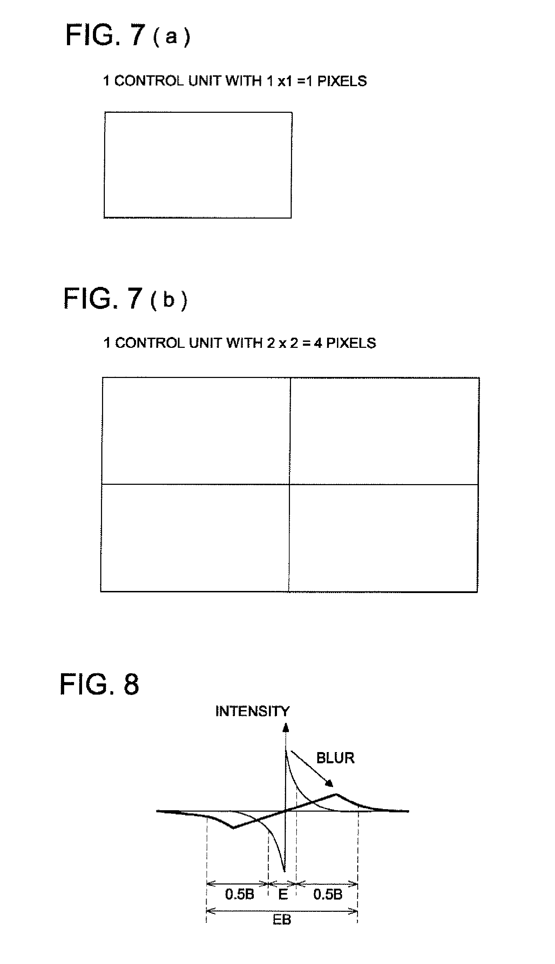

In this case, there are an occasion wherein the minimum control unit is a pixel (Fig. 7 (a)) representing physical resolving power of a digital detector and an image outputting apparatus and an occasion wherein the minimum control unit is an aggregate representing plural pixels (Fig. 7 (b)). For example, pixels in quantity of m x n comprise one control unit, and pixels in quantity of 2 x 2 = 4 comprise one control unit. Output values of respective pixels are averaged to be handled as an output value of the area.

First, detection will be explained. An edge emphasis half-width obtained by a phase contrast imaging method is indicated with EB = E + B as shown in Fig. 8. "E" represents an edge emphasis width formed by X-rays emitted from a point light source whose X-ray source of X-ray tube is ideal. B represents a size of blur caused by geometric unsharpness. Edge emphasis width EB by X-rays emitted from a light source having focal point diameter "D" is broadened by geometric unsharpness to be more than edge emphasis width "E" formed by X-rays emitted from an ideal point light source. EB represents edge emphasis half-width, and it indicates a distance between a peak of the edge and a trough, and is expressed by E + B wherein blur B is added to ideal edge emphasis width "E".

When EB is smaller than a reading pixel size, peaks and troughs of an edge are included in the same pixel to be canceled each other with high probability. It is therefore necessary to conduct image sampling with a reading sampling pitch that is not more than an EB width. Even when the reading pixel size is greater than EB, peaks or troughs of phase contrast edge emphasis are in a different pixel respectively, and the phase contrast edge emphasis is detected. When the reading pixel size is greater than a distance between a peak and a trough, the peak and the trough are located in one pixel to be canceled each other in one occasion, and the peak and the trough are caught in separate pixels, and thereby, phase contrast edge emphasis can be detected in the other occasion. Namely, the phase contrast edge emphasis is detected on a probabilistic basis, and the greater a distance between a peak and a trough is compared with a reading pixel size, the more easily the phase contrast edge emphasis is detected.

For detecting phase contrast edge emphasis with a digital image detector having certain reading sampling pitch "S", it is desirable that a distance between a peak and a trough of the phase contrast edge emphasis is greater than a reading pixel size, namely that the reading sampling pitch "S" is smaller than a distance between a peak and a trough of the phase contrast edge emphasis. Therefore, the condition for detecting the edge accurately is S = E + B.

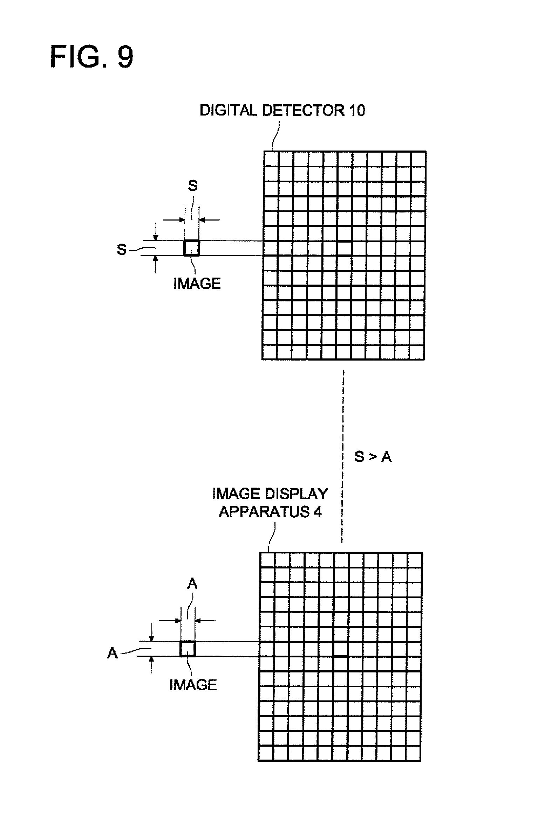

Next, output will be explained. For outputting the detected image information of the edge image without losing it, minimum control unit (reading pixel size or reading sampling pitch) "S" on digital detector 10 and minimum control unit (output pixel size or output writing pitch) "A" of image output apparatus 4 are cause to be in relationship of S > A, as shown in Fig. 9.

By conducting phase contrast imaging, the detected image results in an image that is enlarged from its actual size. In the case of S < A, an image that is suitable for diagnoses cannot be displayed, because the detected image that is greater than the actual size is further enlarged. Therefore, the relationship of S > A is indispensable.

Blur B caused by geometric unsharpness can be obtained by B = D (M - 1). Therefore, the following expression is led from S = E + B.

$$D = (S - E) / (M - 1) \hspace{3cm} (3)$$

Therefore, in the case of EB = E + B, when D = (S - E) / (M - 1) is caused to hold, and when a minimum control unit of digital detector 10 and an aggregate of n pieces of minimum control units of image output apparatus 4 are made to correspond each other as data and minifying interpolation processing (for allotting density values calculated based on an amount of transmitted X-rays for each minimum control unit of digital detector 10, or luminance values, as output data for the aggregate of n pieces) is made to be unnecessary, a problem that an edge emphasis image is lost by the minifying interpolation processing can be solved, and visibility of an edge-emphasized boundary section is improved.

The aggregate of n pieces of minimum control units on the image output apparatus 4 side that causes minimum control units of digital detector 10 to correspond means n = L x L (L is an integer of 1 or more) that has the same number of minimum control units lengthwise and breadthwise when both control units are in a similar figure.

Further, when both control units are not in a similar figure, the aggregate is n = L x L (each of L and P is an integer of 1 or more).

In particular, when "M" represents a magnifying power, it is possible to output image information without losing it, by causing the minifying interpolation processing to be unnecessary, under the conditions of S = MA and n = 1. Owing to this, image deterioration can be prevented, visibility of edge effect is improved, and magnifications agree (life-size outputting will become available).

In the present embodiment, if the phase contrast imaging with magnification of 1.75 is conducted at a reading pixel size of 43.75 $\mu$m, for example, life-size display becomes available (visibility on an edge-emphasized boundary section can naturally be maintained), by causing a reading pixel and an output pixel to correspond to be 1 : 1 without interpolation processing, because of 43.75/25 = 1.75 under the condition that output pixel size is 25 $\mu$m (the visibility of the edge-emphasized boundary section can naturally be maintained).

When the output pixel size is 27 $\mu$m or 23 $\mu$m, if a reading pixel and an output pixel are caused to correspond to be 1 : 1, a magnification for outputting is 1.08 or 0.92, and within this range, X-ray interpretation style is not affected greatly, and diagnoses on a focus area can be conducted accurately by utilizing the visibility on the edge-emphasized boundary section, which is preferable. If the minifying interpolation processing is conducted reluctantly, sticking to the life-size display, on the contrary, edge-emphasized images are lost, and accuracy and efficiency of diagnoses (X-ray interpretation) are lowered.

Though EB = E + B is caused to hold in the present embodiment, most of the edge-emphasized half-widths EB are a blur width on the focal point diameter used in an ordinary medical imaging apparatus, and it is possible to approximate to be EB = B.

If EB = B is caused to hold, S = B holds, and

$$D = S / (M - 1) \hspace{3cm} (4)$$

is led from B = D (M - 1).

Therefore, by causing S > A and D = S / (M - 1) to hold, and by reproducing and outputting minimum control unit of digital detector 10 and an aggregate of n pieces of minimum control units of image outputting apparatus 4 by causing them to correspond as data, digital radiation image imaging system capable of outputting edge-emphasized images is created, image deterioration can be prevented by making the minifying interpolation processing to be unnecessary and visibility of edge effect is improved.

Further, even when approximating to be EB = B, if the conditions include S = MA and n = 1, it is possible to output image information without losing it, by making the minifying interpolation processing to be unnecessary. Due to this, image deterioration can be prevented, visibility of edge effect is improved and magnifications agree each other.

[Example of imaging experiment]

Imaging experiments were conducted for a subject of a columnar plastic fiber having radius r = 0.001 m under the conditions of X-ray energy 50 KeV and an amount of irradiation X-rays of 50 mAs.

An X-ray source for nondestructive inspection wherein a focal point diameter is changeable from 18 $\mu$m to 300 $\mu$m was used as an X-ray source to be used for the image experiment. A target (anode) of an X-ray tube is a tungsten tube. Incidentally, the focal point diameter is not a display size, but is an actual size.

As a digital detector, indirect type FPD (10 cm square) having pixel sizes 100, 140 and 200 $\mu$m was used.

As an image output apparatus, a 18 in. liquid crystal monitor having pixel sizes 50, 70, 100 $\mu$m square, maximum luminance 450 cd/m$^2$ and contrast ratio 800 was used.

Images were displayed without applying image processing such as frequency emphasis processing and gradation processing.

With respect to the evaluation, it was carried out by imaging ACR 156 phantom made by RMI Co. entirely under a condition of the aforesaid focal point diameter, and by observing how No. 5 (0.54 mm nylon fiber) and No. 10 (one set of 6 pieces of 0.24 mm alumina grains) inside the phantom structure can be seen.

Table 1 and Table 2 show typical ones among visual evaluation results for images which were imaged with a focal point diameter of 40 - 300 ($\mu$m) under the evaluation conditions determined based on the aforesaid calculation results.

Table 1 shows an occasion of M = 1.75 (R1 = 1 m, R2 = 0.75 m).

$$E = 24.9 \ (\delta = 8 \times 10^{-7}, \ r = 0.001 \ m)$$

Table 1

| No. | Magnifying power | Focal point diameter D [μm] | Output writing pitch [μm] | Detector reading pitch [μm] | Blur [μm] | Edge-emphasized width EB [μm] | Relationship with expression (3) | Relationship with expression (4) | Judgment |
|---|---|---|---|---|---|---|---|---|---|
| 1 | 1.75 | 40 | 70 | 140 | 30 | 54.9 | Out of range | Out of range | Bad |
| 2 | 1.75 | 80 | 50 | 100 | 60 | 104.9 | Out of range | Out of range | Bad |
| 3 | 1.75 | 160 | 50 | 140 | 120 | 184.9 | Within range | Within range | Passable |
| 4 | 1.75 | 160 | 50 | 140 | 120 | 184.9 | Within range | Out of range | Passable |
| 5 | 1.75 | 160 | 70 | 140 | 120 | 184.9 | Within range | Out of range | Passable |
| 6 | 1.75 | 160 | 100 | 140 | 120 | 184.9 | Out of range | Out of range | Bad |
| 7 | 1.75 | 300 | 70 | 140 | 225 | 324.9 | Within range | Within range | Passable |

Table 2 shows an occasion of M = 2 (R1 = R2 = 1 m).

$$E = 31.5 \ (\delta = 8 \times 10^{-7}, \ r = 0.001 \ m)$$

Table 2

| No. | Magnifying power | Focal point diameter D [$\mu$m] | Output writing reading pitch [$\mu$m] | Detector reading pitch [$\mu$m] | Blur B [$\mu$m] | Edge-emphasized width EB [$\mu$m] | Relationship with expression (3) | Relationship with expression (4) | Judgment |
|---|---|---|---|---|---|---|---|---|---|
| 8 | 2 | 40 | 70 | 140 | 40 | 71.5 | Out of range | Out of range | Bad |
| 9 | 2 | 100 | 50 | 100 | 100 | 131.5 | Within range | within range | Good |
| 10 | 2 | 100 | 70 | 140 | 100 | 131.5 | Out of range | Out of range | Bad |
| 11 | 2 | 160 | 50 | 140 | 160 | 191.5 | Within range | Within range | Good |
| 12 | 2 | 160 | 70 | 140 | 160 | 191.5 | Within range | Within range | Good |
| 13 | 2 | 160 | 100 | 200 | 160 | 191.5 | Out of range | Out of range | Bad |
| 14 | 2 | 300 | 70 | 140 | 300 | 331.5 | Within range | Within range | Passable |
| 15 | 2 | 160 | With inter-polation 50 | 140 | 160 | 191.5 | Within range | Within range | Passable |

An occasion that is within a range of expression (3) $D \geq (S - E) / (M - 1)$ or expression (4) $D \geq S / (M - 1)$ is shown with a term of "WITHIN RANGE", and an occasion that is outside the range is shown with a term of "OUT OF RANGE".

Results of the judgment are shown with "Good" (an edge and a form of the structure can be seen clearly), "Passable" (the structure can be recognized) and with "Bad" (the structure cannot be seen partially).

(a) From the whole of Tables, especially, from Nos. 5 and 6 or from Nos. 12 and 13, it is understood that an edge and a form of the structure can be seen clearly when expression (3) is satisfied, but they cannot be recognized clearly when expression (3) is not satisfied.

(b) From Nos. 1 - 3 or from Nos. 8, 10 and 12, it is understood that images with high image quality can be obtained by large focal point diameter "D" that satisfies expression (3), rather than by small focal point diameter "D".

(c) From the comparison of results between Nos. 4 - 6 and Nos. 11 - 13, it is understood that the trends in (a) are not changed even when the magnifying power is changed.

(d) From the comparison of results between No. 3 and No. 4, it is understood that images with high image quality can be obtained if expression (3) is satisfied, even when "S" representing a reading pixel size or a reading sampling pitch is changed.

(e) From the comparison of results between No. 4 and No. 5, it is understood that images with high image quality can be obtained if expression (3) is satisfied, even when "A" representing an output pixel size or an output writing pitch is changed.

(f) From Nos. 5 and 7 or from Nos. 9, 12 and 14, it is understood that images with high image quality can be obtained if expression (3) is satisfied, even when "D" is changed.

(g) After the comparison between No. 11 and No. 15, No. 11 without interpolation turned out to be of higher judgment. However, No. 11 turned out to be an image that is small in terms of its total size and causes diagnoses to be difficult, when it is compared with No. 15. When No. 11 was enlarged by a magnifier to confirm whether an edge was observed or not, the edge was displayed clearly. For No. 15 with interpolation, interpolation processing is applied for outputting at an actual size. In the same way as in No. 11, a magnifier was used to observe, and an edge was not observed so much.

In the case of a subject on which a boundary form and characteristic differences are not clear like a human body, it is estimated that detection and calculation for edge-emphasized width "E" by X-ray refraction based on a theoretical formula require considerable amount of efforts, which is not preferable.

In this case, a result on the level of "Passable" or higher is obtained from the result by expression (4) that approximates to be EB = B, when expression (4) is satisfied, and it is preferable to use expression (4) practically.

As stated above, in the digital radiation image imaging system of the present embodiment, when reproducing digital image data generated by a phase contrast imaging method on a digital basis, image deterioration is prevented and visibility of edge effect is improved, when a control unit of an imaging system (reading system) and a control unit of a reproducing system are caused to correspond each other so that image data interpolation processing may be made to be unnecessary and image deterioration may be prevented, and imaging conditions in the phase contrast imaging, a reading control unit size and an output control unit size are in the prescribed relationship. Images which can be displayed under the diagnosable state, and have higher sharpness are obtained, and in particular, an improvement in accuracy of image diagnosis is expected in the digital mammography field.

(Second Embodiment)

Fig. 10 shows the structure of digital radiation image imaging system 100 in the present embodiment. Digital radiation image imaging system 100 acquires an enlarged X-ray image of a subject through phase contrast imaging method, and conducts its output.

As shown in Fig. 10, the digital radiation image imaging system 100 is composed of image generating apparatuses 101a - 101d, JOB manager 102, DB (Data Base) 103, image recording apparatuses 104a - 104c and image display apparatuses 104d and 104e. Each apparatus is constructed to be capable of conducting transmission and reception of information through network N conforming to the standards of DICOM (Digital Imaging and Communication in Medicine).

Image generating apparatuses 101a - 101d are those to generate digital data of X-ray images acquired through imaging of a subject, and they are classified into image generating apparatuses 101a and 101b of an imaging system that conducts imaging operations and generating operations for X-ray images on the same apparatus and image generating apparatuses 101c and 101d of a reading system that is constructed separately from the imaging apparatus, and reads X-ray images recording on a portable image detector to generate X-ray image data.

The image generating apparatuses 101a and 101b are equipped with an imaging device composed of an X-ray tube and an image detector (FPD or a phosphor sheet) and with an image generating device that reads X-ray images recorded on the image detector and generates image data, and conduct imaging operations and image generating operations. The image generating device functions as a digital image detector that digitizes X-ray images.

On the other hand, in the case of image generating apparatuses 101c and 101d of the reading system, an image generating device (that functions as a digital image detector) only is provided, and imaging operations are conducted by an imaging apparatus constructed separately by the use of a portable image detector such as a cassette. The image generating apparatuses 101c and 101d conduct reading operations for X-ray images recorded on a cassette through imaging operations.

The phase contrast imaging method and the X-ray image generating method are common to all of the image generating apparatuses 101a - 101d. Detailed explanation for the imaging method will be given later.

JOB manager 102 is one that regulates and controls a flow of X-ray images in digital radiation image imaging system 100. It further conducts output regulation in the case of outputting X-ray images by each outputting apparatus of image recording apparatuses 104a - 104c or of image displaying apparatuses 104d and 104e.

Further, the JOB manager 102 receives information of instruction for imaging designated by a doctor concerning imaging which is called imaging order information from unillustrated HIS (Hospital Information System) or RIS (Radiology Information System), and stores the information. Based on this imaging order information, X-ray images taken through imaging are controlled by the JOB manager 102. For example, since the imaging order information includes patient information (a name, an age and the distinction of sex) concerning a subject (patient) to be imaged and imaging information (a region to be imaged, an imaging direction and an imaging method) concerning imaging, the JOB manager 102 retrieves the imaging order information corresponding to X-ray images, and causes patient information and imaging information included in the imaging order information to accompany the X-ray images. It further causes image generating information (minimum generation unit in the case of image generation, an amount of image data and others) in the course of image generation in image generating apparatuses 101a - 101d to accompany X-ray images. Each X-ray image can be discriminated individually based on accompanying information.

Fig. 11 shows an internal structure of the JOB manager 102.

As shown in Fig. 11, the JOB manager 102 is composed of control section 121, operation section 122, display section 123, communication section and storage section 125.

The control section 121 is composed of CPU (Central Processing Unit) and RAM (Random Access Memory), and it reads out various control programs from the storage section 125, and conducts centralized control for various calculations and operations of respective sections 122 - 125 through cooperation with the program thus read out.

The operation section 122 is equipped with a key board and a mouse, and generates operation signals corresponding to the operations of these operators, and outputs them to the control section 121.

The display section 123 is equipped with a display such as LCD (Liquid Crystal Display), and it displays various types of display information such as various types of operation screens and results of processing by the control section 121.

The communication section 124 is equipped with an interface for communication such as a network interface card, and it conducts transmission and reception of information with each equipment on network N.

The storage section 125 stores data including various types of control programs, parameters necessary for practice of programs, and results of processing by the control section 121.

The storage section 125 further stores output setting table 251.

The output setting table 251 is a table for controlling output setting information in an output apparatus included in digital radiation image imaging system 100, namely, in image recording apparatuses 104a - 104c and image display apparatuses 104d and 104e.

For example, various types of setting information such as a form of output of an output apparatus (film recording or monitor display) and minimum output unit "A" that can be outputted (which is also called minimum control unit in the course of outputting) (unit μm) are stored in the output setting table 251, for each output apparatuses ID (104a - 104d) attached inherently on each output apparatus, as shown in Fig. 12. In this case, the minimum output unit "A" means a minimum constituting unit that constitutes an output image in the case of outputting an X-ray image, and it means a pixel size and a writing pitch size specifically. Incidentally, when a single output apparatus can conduct outputting with plural minimum output units, information of setting plural minimum output units is stored. These pieces of information for setting output are registered and established, each time the output apparatus is introduced newly into digital radiation image imaging system 100.

DB 103 is composed of a large capacity memory, and it stores X-ray images generated through imaging. Each X-ray image is converted into a database by accompanying information that is created by JOB manager 102, to be controlled.

Image recording apparatuses 104a - 104c and image display apparatuses 104d and 104e are those conducting output processing for X-ray images, and the image recording apparatuses 104a - 104c record X-ray images on a film, while, the image display apparatuses 104d and 104e display X-ray images on a monitor. Hereafter, these apparatuses are generically called output apparatuses 104a - 104e.

Each of output apparatuses 104a - 104e has a minimum output unit capable of being outputted. When an X-ray image to be outputted and its output instruction information are inputted by JOB manager 102, output apparatuses 104a - 104e conduct output processing for X-ray images inputted complying with the output instruction information. The output instruction information includes a minimum output unit to be applied in the case of outputting, an output method by that minimum output unit, film sizes and other output conditions. Each of output apparatuses 104a - 104e allots signal values (pixel values) for each minimum generation unit of inputted X-ray image to each minimum generation unit designated complying with a designated output method, and composes an output image composed of minimum output units again, to conduct image output for the outputted image. Incidentally, it is also possible to employ a framework wherein processing to compose output images again is conducted in JOB manager 102 to distribute them to output apparatuses 104a - 104e, and the output apparatuses 104a - 104e conduct only processing to output the output images distributed from the JOB manager 102.

Next, operations of the aforesaid digital radiation image imaging system 100.

With respect to a phase contrast imaging conducted in image generation apparatuses 101a and 101b of the imaging system, it is basically the same as an occasion in the First Embodiment, and an explanation for the phase contrast imaging will be omitted accordingly.

When enlarged image data with high image quality are generated in image generation apparatuses 101a - 101d, image generation information such as minimum generation units "S" (which is also called minimum control unit in the case of reading) and magnifying power "M" are caused to accompany the enlarged image, in each of image generation apparatuses 101a - 101d.

Since reading processing is conducted immediately after imaging and data are generated, in the case of image generation apparatuses 101a and 101b of the imaging system, minimum generation unit "S" and magnifying power "M" are detected automatically on the image generation apparatuses 101a and 101b side, and its information is written on a header area for the enlarged image. With respect to magnifying power "M", it is possible to employ either a framework wherein information of the magnifying power is inputted by a technician, or a framework wherein the magnifying power "M" is automatically calculated in image generation apparatuses 101a and 102b, provided that the framework can detect a subject position and a position of image detector 12 on the image generation apparatuses 101a and 101b side.

Since a cameraman needs to load a cassette on which an enlarged image is recorded in image generation apparatuses 101c and 101d, after imaging, in image generation apparatuses 101c and 101d of a reading system, a framework wherein minimum generation unit "S" and magnifying power "M" are inputted by an operator in that case is employed, and information of the minimum generation unit "S" and magnifying power "M" inputted at image generation apparatuses 101c and 101d are written on a header area of the enlarged image thus read.

Data of enlarged images which are accompanied by image generation information such as minimum generation unit "S" and magnifying power "M" are transmitted to JOB manager 102. In the JOB manager 102, when data of enlarged images are received from image generation apparatuses 101a - 101d, accompanying information based on imaging order information is caused to accompany the enlarged image, and is preserved in DB 103.

After that, in the JOB manager 102, output control processing to distribute enlarged images preserved in DB 103 to output apparatuses 104a - 104e is practiced.

A flow of the output control processing will be explained as follows, referring to Fig. 13. Incidentally, the output control processing is software processing realized by cooperation with processing programs stored in control section 121 and storage section 125.

First, information accompanying the enlarged image data received is referred to, and information of minimum generation unit "S" and magnifying power "M" is acquired (step S1). Then, from these minimum generation unit "S" and magnifying power "M", optimum generation unit Q capable of outputting at a life size is calculated, without conducting minifying interpolation processing. Namely, optimum output unit Q that satisfies the following expression (5) is obtained (step S2).

$$Q = S/M \qquad\qquad (5)$$

When a signal value of minimum generation unit "S" is made to be a signal value of minimum output unit "A" on a basis of one-to-one correspondence between minimum generation unit "S" and minimum output unit "A", minifying interpolation processing turns out to be unnecessary. This also applies to an occasion where a signal value is caused to correspond to aggregate "nA" of minimum output unit "A" (n can take a value of a square of an integer). For example, if magnifying power "M" is 1.75, minimum generation unit "S" is 43.75 ($\mu$m) and minimum output unit "A" is 12.5 ($\mu$m), a single pixel of S = 43.75 ($\mu$m) in the case of image generation results in corresponding to an amount equivalent to 4 pixels (lengthwise: 2 pixels x breadthwise: 2 pixels) each being of A = 12.5 ($\mu$m) on a one-to-one correspondence basis. in the case of outputting.

Therefore, it is possible to output a life-size enlarged image without deteriorating image quality, while keeping edge effects, by conducting output with an output unit (minimum output unit "A" or its aggregate "nA") capable of outputting enlarged images at a size that is the same as or close to the life-size one, even when signal values are allotted as stated above.

Next, output setting table 251 is referred to, and there are selected output apparatuses 104a - 104e wherein minimum output unit "A" can output with an output unit that is desired optimum output unit Q or is closest to the desired optimum output unit Q, and S > A is satisfied (step S3). In this case, an output device of minimum output unit "A" that can output with optimum output unit Q is selected preferentially, and then, the selection is made preferentially, from an output device of minimum output unit "A" that can output with an output unit closest to optimum output unit Q. Incidentally, the relationship of S > A is required by the purpose to output life-size enlarged images. Further, the minimum output unit "A" capable of outputting with optimum output unit Q (or an output unit closest to it) means that an occasion wherein output is possible with minimum output unit "A" itself and an occasion output is possible by aggregate "nA" of minimum output unit "A" are included.

For example, when minimum generation unit "S" is 43.75 ($\mu$m), magnifying power "M" is 1.75 and output to a film is instructed, the optimum output unit Q is obtained from the expression (5) above to be 25 ($\mu$m). In the example of output setting table 251 shown in Fig. 12, there exist two output apparatuses including output apparatuses 104a and 104c, wherein minimum output unit "A" is the same as optimum output unit 25 ($\mu$m) in the case of outputting on a film. Therefore, any one of the output apparatuses 104a and 104c is selected. The selection of the output apparatus may either be on an optional basis, or on other conditions such as a film size that makes it possible for the output apparatus to output.

Then, the selected output apparatuses 104a - 104e are discriminated whether they are in the state to be capable of outputting or not (step S4), and when they are not in the state to be capable of outputting (step S4; N), an output apparatus having minimum output unit "A" that can output with optimum output unit Q or with an output unit closest to the optimum output unit Q is selected (Step S5).

In the aforesaid example, even when the output apparatus 104a among output apparatuses 104a and 104c is selected, if the status information such as that the output apparatus 104a is not turned on or that a large amount of image data to be outputted are waiting for outputting, the output apparatus 104a is judged to be in the state where output is impossible, and an output apparatus that is the same as or is the closest to optimum output unit Q is selected next from other output apparatuses 104b - 104e excluding the output apparatus 104a. In this example, since the output apparatus 104c has minimum output unit "A" (25 $\mu$m) identical to optimum output unit Q, output apparatus 104c is selected.

Further, when both of output apparatuses 104a and 104c each having minimum output unit "A" identical to optimum output unit 25 ($\mu$m) are impossible to output, output apparatus 104b having minimum output unit "A" of 27 ($\mu$m) close next to optimum output unit 25 ($\mu$m) is selected preferentially. If signal values of minimum generation unit s 43.75 ($\mu$m) are allotted to one pixel having a size of minimum output unit "A" (27 ($\mu$m)) on a one-to-one correspondence basis, its output image is one enlarged from a life-size one by 1.08 times as shown in Fig. 14, and it is not a life-size (magnifying power 1.0) one. However, if the magnifying power is as small as this, an image can be used for X-ray interpretation as a life-size one substantially without a problem, and therefore, the selection is made preferentially under the condition that an output close to optimum output unit Q is possible.

In other words, if minimum generation unit "S" is 43.75 ($\mu$m), magnifying power "M" is 1.75 and output is made on a film in the case of output setting shown in Fig. 12, the selection is made preferentially in the order of 25 ($\mu$m) of output apparatus 104a or 104c, 27 ($\mu$m) of output apparatus 104b, 30.2 ($\mu$m) of output apparatus 104c and 43.75 ($\mu$m) of output apparatus 104a.

If output apparatuses 104a - 104c each being capable of outputting are selected as stated above (step S4; Y), output conditions are determined on control section 121, and output instruction information showing the output conditions is generated to be distributed to the aforesaid selected output apparatuses 104a - 104c together with data of enlarged images to be outputted (step S6).

The output condition includes a condition in the case of allotting signal values of minimum generation unit "S" to minimum output unit "A", namely, a condition to allot signal values in one unit of minimum generation unit "S" to one unit of minimum output unit "A" (or its aggregate "nA") by causing them to correspond on a basis of one-to-one correspondence. When allotting with an aggregate unit, information of minimum output unit number n constituting the aggregate "nA" is also included. In output apparatuses 104a - 104e, it is possible to output at a life-size dimension or at a dimension close to the life-size dimension without conducting minifying interpolation processing, because images to be outputted are generated from enlarged images in accordance with the output instruction information, and outputting of them is conducted.

Further, when outputting by plural minimum output units "A" is possible in the selected output apparatuses 104a - 104e, a condition showing which minimum output unit "A" should be used for outputting is included in the output conditions. In addition, if there is a film size or the like designated by a cameraman, the output condition of that size information is also included in the output conditions.

In output apparatuses 104a - 104e where an enlarged image and its output instruction information are received from JOB manager 102, output operations for the enlarged image to be outputted are carried out in accordance with the output instruction information in the aforesaid way.

As stated above, the present embodiment makes it possible to control so that image outputting may be practiced by the output apparatus that conforms to minimum generation unit "S" and magnifying power "M", in spite of a digital radiation image imaging system equipped with plural output apparatuses each having different minimum output unit "A". Owing to this, an enlarged image which is edge-emphasized by phase contrast imaging and has excellent visibility and high image quality can be outputted at a life-size dimension or a dimension close to the life-size dimension, without conducting minifying interpolation processing, and X-ray images which are optimum for X-ray interpretation can be offered.

Further, even when one of output apparatuses 104a - 104e is selected, if the selected one of output apparatuses 104a - 104e is not under the condition to be capable of outputting, selection is made again from other output apparatuses 104a - 104e. Thus, it is possible to distribute images to be outputted, while considering the conditions of plural output apparatuses 104a - 104e.

In addition, when a plurality of minimum output units "A" can be applied in the selected output apparatuses 104a - 104e, it is possible to output of optimum output unit Q by designating the feasible minimum output unit "A".

**Claims**

1. A digital radiation image imaging system, **characterized in that**,
   in the digital radiation image imaging system, which includes: an X-ray tube to irradiate X-rays onto a subject; a digital detector to detect X-rays penetrated through the subject; an image outputting apparatus to output an X-ray image detected by the digital detector, so as to conduct a phase contrast imaging operation,
   a minimum control unit of the digital detector, represented by "S'" ($\mu$m), and a focal point diameter of the X-ray tube, represented by "D" ($\mu$m), fulfill Equations indicated as follows:

$$S > A,$$

   and

$$D \geq (S - E) / (M - 1),$$

   where A ($\mu$m): minimum control unit of the image outputting apparatus,
   R1 (m): distance from a focal point of the X-ray tube to the subject,
   R2 (m): distance from the subject to the digital detector,
   M: magnifying power defined by an Equation of

$$M = (R1 + R2) / R1,$$

   and
   E: edge emphasis width formed by deflections of the X-rays, and
   the minimum control unit "S" of the digital detector and an aggregate of "n" peaces of the minimum control unit "A" of the image outputting apparatus are reproduced and outputted while correlating them with each other as data.

2. A digital radiation image imaging system, **characterized in that**,
   in the digital radiation image imaging system, which includes: an X-ray tube to irradiate X-rays onto a subject; a digital detector to detect X-rays penetrated through the subject; an image outputting apparatus to display an X-ray image detected by the digital detector, so as to conduct a phase contrast imaging operation,
   a minimum control unit of the digital detector, represented by "S" ($\mu$m), and a focal point diameter of the X-ray tube, represented by "D" ($\mu$m), fulfill Equations indicated as follows:

$$S > A,$$

and

$$D \geq S \; / \; (M - 1),$$

where A ($\mu$m): minimum control unit of the image outputting apparatus,
R1 (m): distance from a focal point of the X-ray tube to the subject,
R2 (m): distance from the subject to the digital detector,
M: magnifying power defined by an Equation of

$$M = (R1 + R2) \; / \; R1,$$

and
E: edge emphasis width formed by deflections of the X-rays, and
the minimum control unit "S" of the digital detector and an aggregate of "n" peaces of the minimum control unit "A" of the image outputting apparatus are reproduced and outputted while correlating them with each other as data.

3. The digital radiation image imaging system, recited in claim 1 or 2, **characterized by** further including:

a plurality of image outputting apparatuses;
a selecting means that acquires information of the minimum control unit "A" of each of the plurality of image outputting apparatuses, to select an output means having the minimum control unit "A", which can be outputted in an output unit represented by "S / M" or an output unit nearest to "S / M", among the plurality of image outputting apparatuses; and
an output controlling means that allots a signal value for every minimum generation unit "S" as a signal value for every minimum control unit "A" of the image outputting apparatus selected in the above or every its aggregate "nA", so as to make the image outputting apparatus, selected in the above, conduct an image outputting operation.

4. The digital radiation image imaging system, recited in claim 3, **characterized in that**
the selecting means preferentially selects the image outputting apparatus having the minimum control unit "A", which can be outputted in the output unit nearest to "S / M", next to the other minimum control unit "A", which can be outputted in the output unit represented by "S / M".

5. The digital radiation image imaging system, recited in claim 3 or 4, **characterized in that**,
when one of the plurality of image outputting apparatuses is capable of outputting in a plurality of minimum control units "A", the selecting means preferentially selects the image outputting apparatus having the minimum control unit "A", which can be outputted in the output unit represented by "S / M", from the plurality of minimum control units "A", and the output controlling means makes the image outputting apparatus, selected in the above, conduct an image outputting operation with the minimum control unit "A", which can be outputted in the output unit represented by "S / M", among the plurality of minimum control units "A" possessed by the image outputting apparatus selected.

6. The digital radiation image imaging system, recited in any one of claim 1 - 5, **characterized in that**,
when S = MA, n = 1.

# FIG. 1

PHASE CONTRAST IMAGING APPARATUS (X-RAY IMAGING APPARATUS) 1

IMAGE PROCESSING APPARATUS (WORK STATION) 2

LAN OR WAN

VIEWER 4a

PRINTER 4b

IMAGE OUTPUT APPARATUS 4

PRESERVATION APPARATUS 6

# FIG. 2 (a)

DIGITAL DETECTOR 10

DIGITAL DETECTOR 10

SUBJECT 11

X-RAY TUBE 13

R1

R2

CONTACT IMAGE

FOCAL POINT a

CONTACT IMAGING POSITION

PHASE CONTRAST (ENLARGEMENT) ENLARGEMENT IMAGING POSITION

PHASE CONTRAST (ENLARGEMENT) ENLARGEMENT IMAGING 12

# FIG. 2 (b)

DIGITAL DETECTOR 10

PIXEL

PIXEL

S

S

# FIG. 3

SUBJECT 11

DIGITAL DETECTOR 10

INTENSITY OF X-RAY

RADIUS r

X-RAY POINT LIGHT SOURCE

NON-DENSE

DENSE

R1

R2

PHASE CONTRAST EDGE ENHANCEMENT

20

# FIG. 4

BE : HALF-VALUE WIDTH

INTENSITY

MAXIMUM (PEAK)
MAXIMUM VALUE 50%

MINIMUM VALUE 50%
MINIMUM (TROUGH)

1

# FIG. 5

COOLIDGE
X-RAY TUBE 5

SUBJECT 11

DIGITAL DETECTOR 10

X-RAY
INTENSITY

FOCUS
DIAMETER D

R1

R2

BLUR : B

HALF-VALUE WIDTH
BROADENED BY BLUR

PHASE CONTRAST
EDGE ENHANCEMENT

# FIG. 6

X-RAY INTENSITY

BLUR CAUSED BY
GEOMETRIC
UNSHARPNESS

BLUR CAUSED BY
GEOMETRIC
UNSHARPNESS

# FIG. 7 ( a )

1 CONTROL UNIT WITH 1 x1 =1 PIXELS

# FIG. 7 ( b )

1 CONTROL UNIT WITH 2 x 2 = 4 PIXELS

# FIG. 8

INTENSITY

BLUR

0.5B    E    0.5B

EB

# FIG. 9

DIGITAL DETECTOR 10

S

S

IMAGE

S > A

IMAGE DISPLAY
APPARATUS 4

A

A

IMAGE

# FIG. 10

# FIG. 11

102

CONTROL
SECTION ~121

STORAGE
SECTION ~125

OUTPUT
SETTING
TABLE ~251

122~ OPERATION
SECTION

123~ DISPLAY SECTION

124~ COMMUNICATION
SECTION

# FIG. 12

251

| OUTPUT APPARATUS ID | OUTPUT FORM | MINIMUM OUTPUT UNIT (μm) | |
|---|---|---|---|
| 104a | FILM RECORDING | 25 | 43.75 |
| 104b | FILM RECORDING | 27 | ——— |
| 104c | FILM RECORDING | 25 | 30.2 |
| 104d | MONITOR DISPLAY | 30 | ——— |
| 104e | MONITOR DISPLAY | 100 | ——— |

# FIG. 13

```
                    ┌──────────────┐
                    │    START     │
                    └──────┬───────┘
                           │
                           ▼
        ┌─────────────────────────────────┐
        │ ACQUISITION OF INFORMATION      │
        │ OF MINIMUM GENERATION UNIT S    │ ── S1
        │ OF ENLARGED IMAGE AND           │
        │ MAGNIFICATION RATE M            │
        └─────────────────┬───────────────┘
                          │
                          ▼
        ┌─────────────────────────────────┐
        │ CALCULATION OF OUTPUT UNIT Q    │
        │ CAPABLE OF OUTPUTTING AT LIFE   │
        │ SIZE FROM MINIMUM GENERATION    │ ── S2
        │ UNIT S AND MAGNIFICATION RATE M,│
        │ WITHOUT CONDUCTING REDUCTION    │
        │ INTERPOLATION PROCESSING        │
        └─────────────────┬───────────────┘
                          │
                          ▼
        ┌─────────────────────────────────┐
        │ SELECTING OUTPUT APPARATUS      │
        │ HAVING OPTIMUM OUTPUT UNIT Q    │
        │ OR MINIMUM OUTPUT UNIT A        │ ── S3
        │ CLOSEST TO OPTIMUM OUTPUT       │
        │ UNIT Q, REFERRING TO OUTPUT     │
        │ SETTING TABLE                   │
        └─────────────────┬───────────────┘
                          │
                          ▼         S4
                    ◇─────────────◇        N
                   ╱ IS SELECTED   ╲ ──────────┐
                  ⟨ OUTPUT APPARATUS ⟩          │
                   ╲ READY TO      ╱            │   S5
                    ◇  OUTPUT?    ◇             ▼
                          │ Y        ┌─────────────────────┐
                          │          │ SELECTING OUTPUT    │
                          │          │ APPARATUS HAVING    │
                          ▼          │ OPTIMUM OUTPUT UNIT Q│
        ┌─────────────────────────┐  │ OR MINIMUM OUTPUT   │
        │ DETERMINING OUTPUT      │  │ UNIT A CLOSEST TO   │
        │ CONDITIONS AND          │  │ OPTIMUM OUTPUT UNIT Q│
        │ DISTRIBUTING THEM TO    │  └─────────────────────┘
        │ SELECTED OUTPUT         │ ── S6
        │ APPARATUS AS OUTPUT     │
        │ INSTRUCTION INFORMATION │
        │ TOGETHER WITH ENLARGED  │
        │ IMAGE TO BE OUTPUTTED   │
        └─────────────┬───────────┘
                      │
                      ▼
              ┌──────────────┐
              │     END      │
              └──────────────┘
```

# FIG. 14

MINIMUM OUTPUT UNIT A
25μm

OUTPUT IMAGE
(ENLARGEMENT RATE 1)
LIFE SIZE

MINIMUM GENERATION UNIT S
43.75μm

ENLARGED IMAGE
ENLARGEMENT RATE 1.75

ALLOCATING SIGNAL
VALUES WITH 1 : 1

MINIMUM OUTPUT UNIT A
27.0μm

OUTPUT IMAGE
ENLARGEMENT RATE 1.08

EP 1 886 629 A1

## INTERNATIONAL SEARCH REPORT

| International application No. |
| --- |
| PCT/JP2006/309544 |

**A. CLASSIFICATION OF SUBJECT MATTER**
*A61B6/00*(2006.01)

According to International Patent Classification (IPC) or to both national classification and IPC

**B. FIELDS SEARCHED**

Minimum documentation searched (classification system followed by classification symbols)
*A61B6/00*(2006.01)-*A61B6/00*(2006.01)

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2003-180670 A (Konica Corp.), 02 July, 2003 (02.07.03), Full text; Figs. 1 to 10 & US 2003/0123611 A1 | 1-6 |
| Y | JP 2004-208773 A (Konica Minolta Holdings Kabushiki Kaisha), 29 July, 2004 (29.07.04), Full text; Figs. 1 to 4 & US 7027556 B2 | 1-6 |

| ☐ Further documents are listed in the continuation of Box C. | ☐ See patent family annex. |
| --- | --- |

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 23 August, 2006 (23.08.06) | 05 September, 2006 (05.09.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001311701 A